(19) **Europäisches Patentamt**

**European Patent Office**

**Office européen des brevets**

(11) **EP 1 582 148 A1**

(12) **EUROPÄISCHE PATENTANMELDUNG**

(43) Veröffentlichungstag:
**05.10.2005 Patentblatt 2005/40**

(51) Int Cl.⁷: **A61B 6/06**, A61B 6/00, G21K 3/00

(21) Anmeldenummer: **05101998.2**

(22) Anmeldetag: **15.03.2005**

| | |
|---|---|
| (84) Benannte Vertragsstaaten:<br>**AT BE BG CH CY CZ DE DK EE ES FI FR GB GR HU IE IS IT LI LT LU MC NL PL PT RO SE SI SK TR**<br>Benannte Erstreckungsstaaten:<br>**AL BA HR LV MK YU** | (71) Anmelder: **SIEMENS AKTIENGESELLSCHAFT**<br>**80333 München (DE)** |
| (30) Priorität: **31.03.2004 DE 102004015860** | (72) Erfinder: **Bernhardt, Philipp**<br>**91301, Forchheim (DE)** |

(54) **Röntgenvorrichtung für die Mammographie**

(57) Die Erfindung betrifft eine Röntgenvorrichtung für die Mammographie, mit einer eine aus Wolfram hergestellte Anode aufweisenden Röntgenröhre, einem in Strahlrichtung der Röntgenröhre nachgeordneten Filter und einem dem Filter nachgeordneten, aus einem halbleitenden Material hergestellten Detektor. Zur Verbesserung der Qualität mammographischer Röntgenaufnahmen sowie zur gleichzeitigen Reduzierung der Strahlendosis wird erfindungsgemäß vorgeschlagen, dass das Filter aus einem Filtermaterial hergestellt ist, dessen K-Absorptionskante im Bereich zwischen 3,8 keV und 7,3 keV liegt.

FIG 1

EP 1 582 148 A1

**Beschreibung**

Die Erfindung betrifft eine Röntgenvorrichtung für die Mammographie nach dem Oberbegriff des Anspruchs 1.

Eine solche Röntgenvorrichtung ist z. B. aus Michael Flynn, Charles Dodge, Donald Peck, Ann Swinford, "Optimal radiographic techniques for digital mammograms obtained with an amorphous selenium detector", Proceedings of SPIE Vol. 5030 (2003) bekannt. Basierend auf Computersimulationen werden in dieser Arbeit vier willkürlich ausgewählte Filtermaterialien, Ag, Al, Mo, Rh und Sn verglichen. Dabei erweisen sich aus Ag und Sn hergestellte Filter als günstig. Für Sn war eine von einem Patienten absorbierte Strahlendosis am kleinsten. Trotz reduzierter Strahlendosis können mammographische Untersuchungen Strahlenschäden verursachen, so dass eine weitere Reduktion der Strahlendosis erstrebenswert ist.

Aufgabe der Erfindung ist es, die Nachteile nach dem Stand der Technik zu beseitigen. Es sollen alternative Filtermaterialien angegeben werden, mit welchen eine von einem Patienten absorbierte Strahlendosis bei gleichbleibender oder verbesserter Qualität mammographischer Röntgenaufnahmen weiter verringert werden kann.

Diese Aufgabe wird durch die Merkmale des Anspruchs 1 gelöst. Zweckmäßige Ausgestaltungen ergeben sich aus den Ansprüchen 2 bis 5.

Nach Maßgabe der Erfindung ist vorgesehen, dass das Filter aus einem Filtermaterial hergestellt ist, dessen K-Absorptionskante im Bereich zwischen 3,8 keV und 7,3 keV liegt. - Derartige Filter werden nachfolgend mit K-Filter bezeichnet. Allgemein wird Röntgenstrahlung mit Quantenenergien kleiner als etwa 15 keV vom Gewebe stark absorbiert. K-Filter besitzen für diese Quantenenergien im Mittel deutlich höhere atomare Wirkungsquerschnitte als für höherenergetische Quantenenergien im Bereich von etwa 15 - 45 keV. Damit kann die vom Gewebe stark absorbierte Röntgenstrahlung und eine damit verbundene Strahlenbelastung reduziert werden.

Mittels Mammographie können krankhafte Veränderungen im Gewebe einer Brust, wie z. B. Kalzifikationen oder Tumore, nachgewiesen werden. Aufgrund unterschiedlicher Absorptionseigenschaften, wie z. B. Dichte oder Dicke, muss die Röntgenstrahlung an das jeweilige Gewebe angepasst werden. Das ist bei Verwenden eines K-Filters möglich. Eine durch ein K-Filter transmittierte Röntgenstrahlung mit einer maximalen Quantenenergie kleiner als 45 keV besitzt ein kontinuierliches Spektrum mit einem Maximum. Durch Verändern der maximalen Quantenenergie oder der Dicke des Filters kann das Maximum zu höheren oder niedrigeren Werten verschoben werden, und zwar insbesondere in einen für die Mammographie günstigen Bereich zwischen 15 und 45 keV. Damit ist es möglich, mammographische Röntgenaufnahmen qualitativ zu optimieren. Beispielsweise lassen sich der Kontrast und das Signal zu Rauschverhältnis maximieren.

Nach einer weiteren Ausgestaltung der Erfindung wird die Röntgenröhre mit einer Spitzenspannung zwischen 15 und 45 kVp betrieben. Die damit erzeugte Röntgenstrahlung besitzt eine für qualitativ hochwertige mammographische Röntgenaufnahmen besonders vorteilhafte maximale Quantenenergie zwischen 15 und 45 keV.

Nach einer weiteren Ausgestaltung der Erfindung ist das halbleitende Material des Detektors aus Selen hergestellt. Ein solcher Detektor wird in herkömmlichen Röntgenvorrichtungen für die Mammographie eingesetzt. Er ist kommerziell erhältlich.

Nach einer vorteilhaften Ausgestaltung ist das Filtermaterial aus folgender Gruppe ausgewählt: Ca, Sc, Ti, V, Cr, Mn, Fe. Die vorgeschlagenen Filtermaterialien absorbieren Röntgenquanten mit einer Energie kleiner als etwa 15 keV besonders stark. Röntgenquanten mit Energien größer als etwa 15 keV werden weniger stark absorbiert. Obige Filtermaterialien besitzen Ordnungszahlen zwischen 20 und 26. Für diese Ordnungszahlen wird eine Absorption von Röntgenstrahlung mit einer Energie im Bereich von etwa 15 - 45 keV hauptsächlich durch einen im Filtermaterial auftretenden Photoeffekt verursacht. Der Photoeffekt verursacht keine Streustrahlung, welche zu einer von einem Patienten absorbierten Dosis beitragen kann.

Mit den obigen Filtermaterialien ist es möglich, die Strahlenbelastung, z. B. durch Wahl einer geeigneten Absorptionsdicke des Filters, zu kontrollieren. Des Weiteren kann die durch das Filter transmittierte Röntgenstrahlung an die Absorptionseigenschaften des Gewebes, z. B. durch eine geeignete Wahl der maximalen Quantenenergie, angepasst werden. Unter Verwendung der vorgenannten Filtermaterialien können mammographische Röntgenaufnahmen mit verbesserter Qualität bei einer verringerten Strahlendosis hergestellt werden.

Nach einer weiteren Ausgestaltung der Erfindung weist das Filter in Strahlrichtung eine Dicke im Bereich von 0,05 bis 1 mm auf. Mit der vorgeschlagenen Dicke kann eine für die Mammographie besonders geeignete Strahlendosis erzeugt werden.

Nachfolgend wird die Erfindung anhand der Figuren näher erläutert. Es zeigen:

Fig. 1    atomare Wirkungsquerschnitte,

Fig. 2a    Qualitätsfaktoren für Mikrokalzifikationen,

Fig. 2b    Qualitätsfaktoren für Tumore und

Fig. 3    Intensitätsverteilungen.

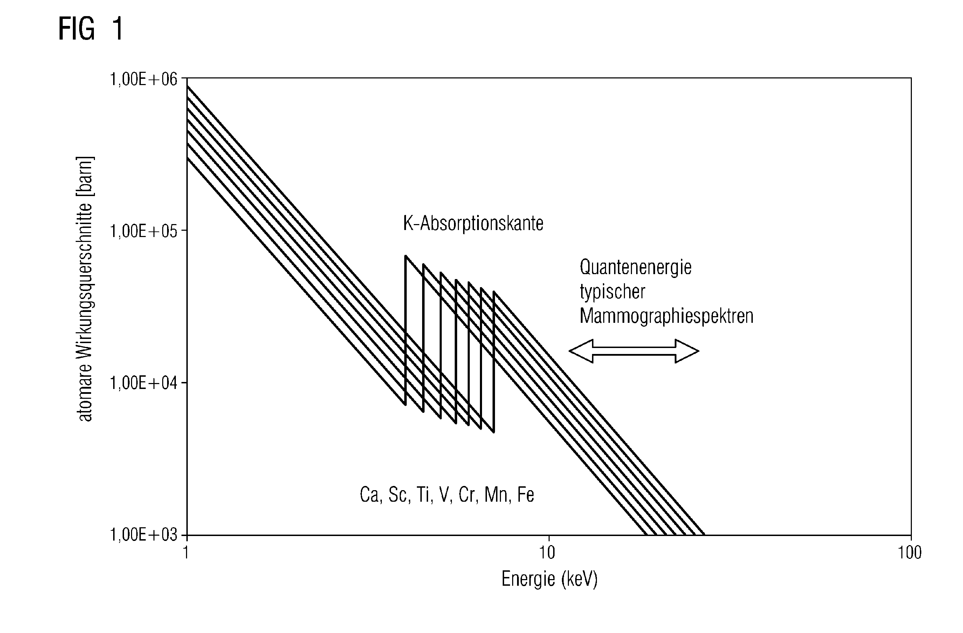

In Figur 1 sind atomare Wirkungsquerschnitte der Elemente Ca, Sc, Ti, V, Cr, Mn, Fe für Röntgenstrahlung mit Quantenenergien im Bereich von 1 bis etwa 45 keV dargestellt. Mittels eines Doppelpfeiles ist der für die Mammographie typische Bereich von Quantenenergien von etwa 15 bis 45 keV bezeichnet. Die K-Absorptionskanten liegen zwischen 4.0 und 7.1 keV. Bei den K-Absorptionskanten steigt der atomare Wirkungsquerschnitt um etwa eine Größenordnung an. Für Röntgenstrahlen mit für die Mammographie typischen Quantenenergien sind die atomaren Wirkungsquerschnitte im Mittel deutlich kleiner als für Röntgenstrahlen mit Quantenenergien kleiner als etwa 15 keV.

Die in Figur 1 gezeigten Elemente absorbieren insbesondere Quantenenergien kleiner als etwa 15 keV. Solche Quantenenergien werden im Gewebe stark absorbiert und tragen wesentlich zu einer von einem Patienten absorbierten Strahlendosis bei. Durch Filterung können diese Quantenenergien weitgehend unterdrückt werden. Quantenenergien im Bereich zwischen etwa 15 und 45 keV sind bei der Mammographie für qualitativ gute Röntgenaufnahmen besonders wichtig. Sie werden durch ein aus obigen Materialien hergestelltes Filter gut transmittiert. Damit ist es möglich, bei minimaler Strahlendosis eine maximale Qualität der Röntgenaufnahmen zu erzielen.

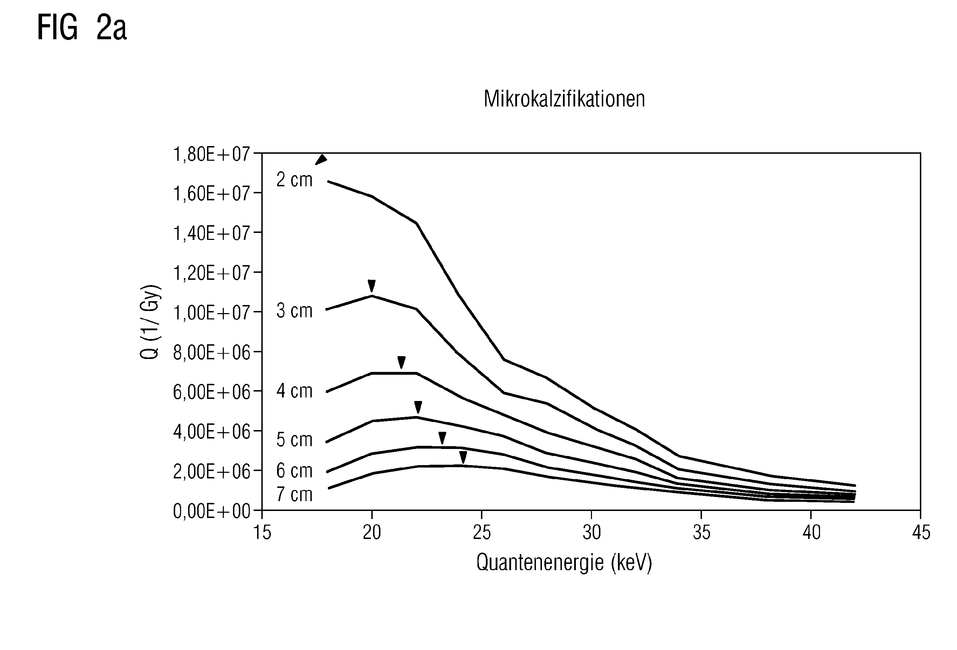

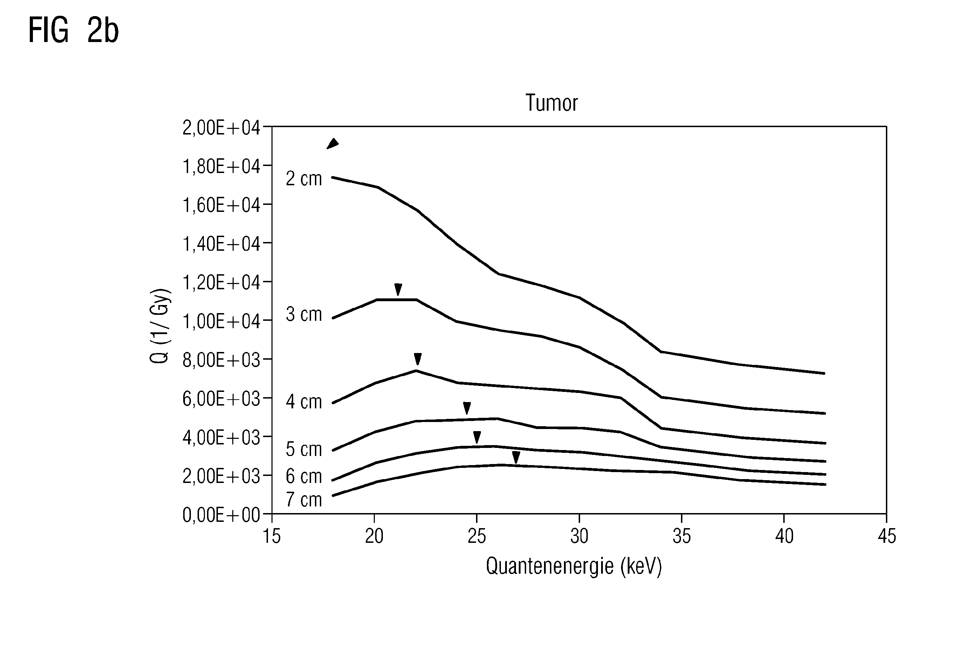

In Figuren 2a bzw. 2b sind simulierte Qualitätsfaktoren mammographischer Röntgenaufnahmen gegen Quantenenergien aufgetragen. Es sind jeweils 2, 3, 4, 5, 6, und 7 cm dicke Gewebe mit Mikrokalzifikationen bzw. Tumoren berücksichtigt. Die Quantenenergien liegen im Bereich von 18 bis 42 keV. Die Qualitätsfaktoren sind wie üblich definiert:

$$Q = (CNR)^2 / D,$$

wobei CNR als Kontrast zu Rauschverhältnis definiert ist und D eine vom Gewebe absorbierte, gemittelte Strahlendosis ist. Die Qualitätsfaktoren wurden unter folgenden Annahmen simuliert:

- Das Gewebe besteht aus 50% Fett- (Ap6) und 50% Drüsengewebe (BR12),
- BR12 enthält 54,6% H; 36,9% C; 1,07% N; 7,1% O; 0,0251% Cl; 0,15% Ca mit einer Dichte von 0,97g/cm3,
- AP6 enthält 53,7% H; 37,2% C; 1,09% N; 6,85% O; 1,04 F; 0,0256% Cl, mit einer Dichte von 0,92g/cm3,
- Mikrokalzifikationen und Tumore haben eine zylindrische Form, mit einer zur Röntgenstrahlung senkrechten, zentralen Achse, und sind mittig im Gewebe angeordnet,
- Mikrokalzifikationen bestehen aus Ca5 P3 O13 H, weisen eine Dichte von 3,2 g/cm3 und einen Radius von 0,1 mm auf,
- Tumore bestehen aus BR12, weisen eine Dichte von 1,044 g/cm3 und einen Radius von 2,5 mm auf
- die Röntgenstrahlung besitzt einen Fokus der Größe 0,3 mm,

- die Energie pro Röntgenaufnahme beträgt 2,5 kWs,
- Röntgenstrahlung wird mit 1,0 mm Be vorgefiltert,
- der Abstand Fokus - Detektor beträgt 650 mm,
- der Abstand Gewebe - Detektor beträgt 40 mm,
- Detektion mittels eines Se-Detektors mit einer Absorptionsdicke von 0,25 mm, einer Dichte von 4,28 g/cm3, einer Pixelgröße von 0,07 mm, einem Füllfaktor von 1,00, und einer Pixelanzahl von 4096 x 3584).

In den Fig. 2a und 2b sind die Maxima der Qualitätsfaktoren durch Dreiecke gekennzeichnet. Bei der zu einem Maximum gehörenden Quantenenergie ist D minimal und CNR maximal. Bei größerer Dicke des Gewebes liegt das Maximum bei höheren Quantenenergien. Des Weiteren sind die Maxima davon abhängig, ob im Gewebe Kalzifikationen oder Tumore enthalten sind. Bei gleicher Gewebedicke liegt das Maximum für Tumore bei höheren Quantenenergien als das für Kalzifikationen. Figuren 2a und 2b zeigen, dass für qualitativ hochwertige mammographische Röntgenaufnahmen eine Anpassung der Röntgenstrahlung an Gegebenheiten des Gewebes erforderlich ist.

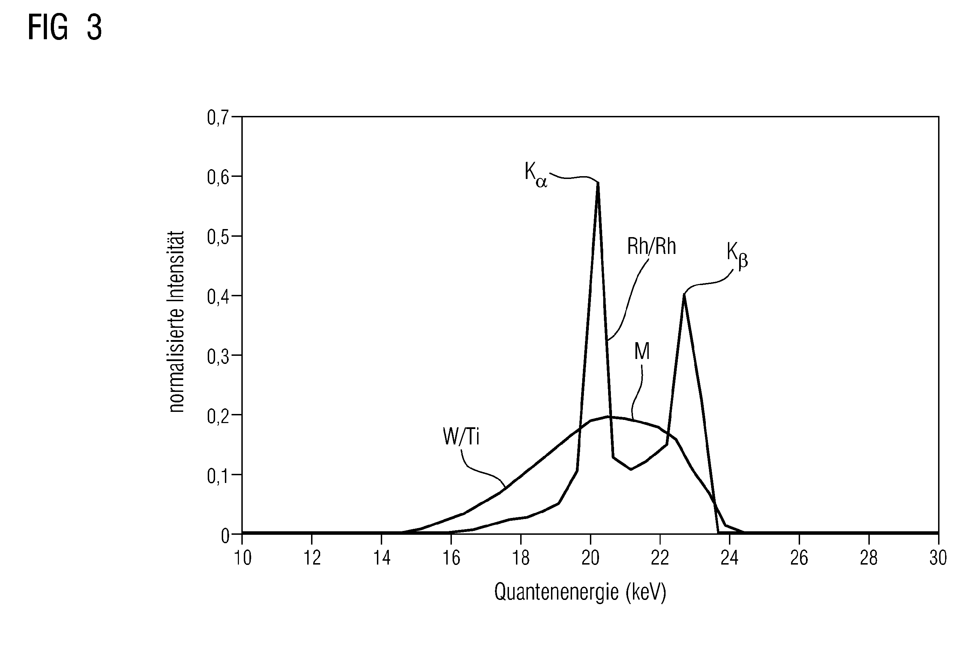

Figur 3 zeigt normierte Intensitätsverteilungen von Röntgenstrahlung. Diese wurden gemäß J. Boone, "Molybdenium, rhodium and tungsten anode spectral models using interpolating polynomials with application to mammography", Med. Phys. 24(12), 1863-1874 (1997) berechnet. W/Ti bezeichnet eine Intensitätsverteilung bei Verwendung einer Wolframanode in Kombination mit einem 0,4 mm dicken, aus Ti hergestellten Filter und bei einer Spitzenspannung von 24 kVp. Rh/Rh bezeichnet eine Intensitätsverteilung für eine Kombination aus einer aus Rh hergestellten Anode und einem aus Rh hergestellten, 0,18 mm dicken Filter, bei einer Spitzenspannung von 34 kVp.

Rh/Rh wird von zwei charakteristischen Röntgenlinien bei etwa 20,0 keV $K_\alpha$, und 23,2 keV $K_\beta$ geprägt. W/Ti ist dagegen kontinuierlich ausgebildet und weist ein Maximum M bei einer für die Detektion von Kalzifikationen optimalen Quantenenergie von etwa 21 keV auf. Während bei Rh/Rh die charakteristischen Röntgenlinien $K_\alpha$ und $K_\beta$ in ihrer Lage unveränderlich sind, kann das Maximum M bei W/Ti durch Verändern der Spitzenspannung oder durch Wahl einer geeigneten Dicke des Filters zu kleineren oder größeren Quantenenergien hin verschoben werden. Bei einer optimal an ein zu untersuchendes Gewebe angepassten Intensitätsverteilung liegt das Maximum M der jeweiligen Quantenenergie dort, wo die Qualitätsfaktoren gemäß Fig.2a oder 2b maximal sind. Die mit dem W/Ti-System erzeugte Intensitätsverteilung kann flexibel angepasst werden, so dass größere Qualitätsfaktoren erreicht werden können.

Das W/Ti-System ist besonders vorteilhaft für Quantenenergien > etwa 25 keV. Beispielsweise kann damit bei einer Detektion eines Tumors, mit CNR = 1,00 die Strahlendosis gegenüber einem Rh/Rh-System um 44% verringert werden.

**Patentansprüche**

1.  Röntgenvorrichtung für die Mammographie, mit einer eine aus Wolfram hergestellte Anode aufweisenden Röntgenröhre, einem in Strahlrichtung der Röntgenröhre nachgeordneten Filter und einem dem Filter nachgeordneten, aus einem halbleitenden Material hergestellten Detektor,
    **dadurch gekennzeichnet , dass**
    das Filter aus einem Filtermaterial hergestellt ist, dessen K-Absorptionskante im Bereich zwischen 3,8 keV und 7,3 keV liegt.

2.  Röntgenvorrichtung nach Anspruch 1, wobei die Röntgenröhre mit einer Spitzenspannung zwischen 15 und 45 kVp betrieben wird.

3.  Röntgenvorrichtung nach einem der vorherigen Ansprüche, wobei das halbleitende Material des Detektors aus Selen hergestellt ist.

4.  Röntgenvorrichtung nach einem der vorherigen Ansprüche, wobei das Filtermaterial aus folgender Gruppe ausgewählt ist: Ca, Sc, Ti, V, Cr, Mn, Fe.

5.  Röntgenvorrichtung nach einem der vorherigen Ansprüche, wobei das Filter in Strahlrichtung eine Dikke im Bereich von 0,05 bis 1 mm aufweist.

FIG 1

atomare Wirkungsquerschnitte [barn] vs. Energie (keV)

K-Absorptionskante

Quantenenergie typischer Mammographiespektren

Ca, Sc, Ti, V, Cr, Mn, Fe

FIG 2a

Mikrokalzifikationen

FIG 2b

EP 1 582 148 A1

FIG 3

EP 1 582 148 A1

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 2003/227996 A1 (FRANCKE TOM ET AL) 11. Dezember 2003 (2003-12-11) | 1,2,4,5 | A61B6/06 A61B6/00 G21K3/00 |
| Y | * Absätze [0037] - [0044] * * Absätze [0088] - [0090] * * Abbildungen 2,5 * ----- | 3 | |
| Y,D | FLYNN M J ET AL: "Optimal radiographic techniques for digital mammograms obtained with an amorphous selenium detector" MEDICAL IMAGING 2003. PHYSICS OF MEDICAL IMAGING 16-18 FEB. 2003 SAN DIEGO, CA, USA, Bd. 5030, Juni 2003 (2003-06), Seiten 147-156, XP002331371 Proceedings of the SPIE - The International Society for Optical Engineering SPIE-Int. Soc. Opt. Eng USA ISSN: 0277-786X * Seite 147, letzter Absatz - Seite 148, Absatz 3 * ----- | 3 | |
| A | MAUDAL S: "Iron filters as a means of reducing the dose in roentgen examination of the female breast." ACTA RADIOLOGICA: THERAPY, PHYSICS, BIOLOGY. JUN 1968, Bd. 7, Nr. 3, Juni 1968 (1968-06), Seiten 238-240, XP008048376 ISSN: 0567-8064 * das ganze Dokument * ----- | 1,2,4 | **RECHERCHIERTE SACHGEBIETE** (Int.Cl.7) A61B G21K |
| A | DE 42 04 301 A1 (SIEMENS AG, 8000 MUENCHEN, DE) 11. Februar 1993 (1993-02-11) * Seite 1, Zeile 6 - Seite 2, Zeile 1 * ----- -/-- | 1,2,4 | |

Der vorliegende Recherchenbericht wurde für alle Patentansprüche erstellt

| Recherchenort | Abschlußdatum der Recherche | Prüfer |
|---|---|---|
| Berlin | 10. Juni 2005 | Völlinger, M |

**Europäisches Patentamt**

**EUROPÄISCHER RECHERCHENBERICHT**

Nummer der Anmeldung

EP 05 10 1998

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | DATABASE WPI<br>Section Ch, Week 200028<br>Derwent Publications Ltd., London, GB;<br>Class K08, AN 2000-318642<br>XP002331372<br>& CN 1 243 320 A (UNIV SHANDONG MEDICAL)<br>2. Februar 2000 (2000-02-02)<br>* Zusammenfassung *<br>----- | 1,4 | |
| | | | **RECHERCHIERTE SACHGEBIETE (Int.Cl.7)** |

Der vorliegende Recherchenbericht wurde für alle Patentansprüche erstellt

| Recherchenort | Abschlußdatum der Recherche | Prüfer |
|---|---|---|
| Berlin | 10. Juni 2005 | Völlinger, M |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 05 10 1998

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-06-2005

| Im Recherchenbericht angeführtes Patentdokument | Datum der Veröffentlichung | Mitglied(er) der Patentfamilie | Datum der Veröffentlichung |
|---|---|---|---|
| US 2003227996 A1 | 11-12-2003 | SE 524731 C2<br>AU 2003241247 A1<br>CA 2484945 A1<br>SE 0201742 A<br>WO 03103495 A1 | 21-09-2004<br>22-12-2003<br>18-12-2003<br>08-12-2003<br>18-12-2003 |
| DE 4204301 A1 | 11-02-1993 | CH 683728 A5<br>JP 3270129 B2<br>JP 5211051 A | 29-04-1994<br>02-04-2002<br>20-08-1993 |
| CN 1243320 A | 02-02-2000 | KEINE | |

Für nähere Einzelheiten zu diesem Anhang : siehe Amtsblatt des Europäischen Patentamts, Nr.12/82

EPO FORM P0461